(19) Europäisches Patentamt
European Patent Office
Office européen des brevets

(11) EP 1 068 864 B1

(12) FASCICULE DE BREVET EUROPEEN

(45) Date de publication et mention de la délivrance du brevet: **09.02.2005 Bulletin 2005/06**

(51) Int Cl.⁷: **A61K 7/48**

(21) Numéro de dépôt: **00401742.2**

(22) Date de dépôt: **19.06.2000**

# (54) Utilisation d'au moins un hydroxystilbène comme agent anti-glycation.

Anwendung von mindestens einem Hydroxystilben als Glykationshemmer

Use of at least one hydroxystilbene as glycation inhibitor

(84) Etats contractants désignés:
**AT BE CH CY DE DK ES FI FR GB GR IE IT LI LU MC NL PT SE**

(30) Priorité: **16.07.1999 FR 9909267**

(43) Date de publication de la demande:
**17.01.2001 Bulletin 2001/03**

(73) Titulaire: **L'OREAL**
**75008 Paris (FR)**

(72) Inventeurs:
- **Liviero, Christel**
  **75008 Paris (FR)**
- **Breton, Lionel**
  **78000 Versailles (FR)**
- **Pageon, Hervé**
  **92800 Puteaux (FR)**

(74) Mandataire: **Galup, Cédric Olivier Nicolas**
**L'OREAL**
**Départment Propriété Industrielle**
**6, rue Bertrand Sincholle**
**92585 Clichy Cedex (FR)**

(56) Documents cités:
**FR-A- 2 777 183** **FR-A- 2 777 184**
**FR-A- 2 777 186**

- **PATENT ABSTRACTS OF JAPAN vol. 013, no. 227 (C-600), 25 mai 1989 (1989-05-25) & JP 01 038009 A (POLA CHEM IND INC), 8 février 1989 (1989-02-08)**
- **PATENT ABSTRACTS OF JAPAN vol. 1996, no. 11, 29 novembre 1996 (1996-11-29) & JP 08 175960 A (KAO CORP), 9 juillet 1996 (1996-07-09)**
- **PATENT ABSTRACTS OF JAPAN vol. 015, no. 297 (C-0854), 29 juillet 1991 (1991-07-29) & JP 03 109343 A (MARUZEN KASEI CO LTD), 9 mai 1991 (1991-05-09)**
- **PATENT ABSTRACTS OF JAPAN vol. 1998, no. 04, 31 mars 1998 (1998-03-31) & JP 09 328410 A (YUSHIRO CHEM IND CO LTD;MITSUBA BOEKI KK), 22 décembre 1997 (1997-12-22)**
- **PATENT ABSTRACTS OF JAPAN vol. 1998, no. 06, 30 avril 1998 (1998-04-30) & JP 10 045566 A (YUSHIRO CHEM IND CO LTD;MITSUBA BOEKI KK), 17 février 1998 (1998-02-17)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 068 864 B1

## Description

L'invention se rapporte à l'utilisation d'au moins un hydroxystilbène de formule (I)

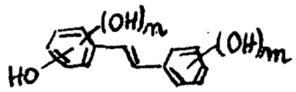

dans laquelle n est un nombre entier compris entre 0 et 4 inclusivement et m est un nombre entier compris entre 0 et 5 inclusivement dans une composition cosmétique, l'hydroxystilbène ou la composition étant destinés à diminuer voire inhiber la glycation des protéines, particulièrement des protéines de la peau et/ou de ses annexes.

La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine.

Ce phénomène augmente de façon régulière avec l'âge. Il se caractérise par l'apparition de produits de glycation dont la teneur augmente de façon régulière en fonction de l'age. Les produits de glycation sont par exemple la pyrraline, la carboxyméthyl-lysine, la pentosidine, la crossline, la $N^{\varepsilon}$(2-carboxyéthyl)-lysine (CEL), la glyoxal-lysine dimer (GOLD), la méthylglyoxal-lysine dimer (MOLD), la 3DG-ARG imidazolone, les versperlysines A, B, C, la thréosidine ou encore les produits finaux de glycosylation avancée (advanced glycosylation ends products ou AGEs).

La glycation des protéines est donc un phénomène universel, bien connu au niveau de la peau, particulièrement au niveau de sa composante dermique, ; mais qui survient également dans les annexes de celle-ci comme les ongles ou les cheveux, particulièrement sur les kératines et plus généralement dans tout système protéique pour peu que les conditions requises pour la glycation soient réunies.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même de différentes protéines extracellulaires parmi lesquelles figure notamment les fibres de collagène, l'élastine et différentes glycoprotéines. L'ensemble de ces composants extracellulaires est synthétisé par le fibroblaste. On trouve également dans le derme des leucocytes, des mastocytes ou encore des macrophages tissulaires. Enfin le derme contient des vaisseaux sanguins et des fibres nerveuses.

Le fibroblaste, de par son activité dans la synthèse des protéines extracellulaires matricielles (protéoglycannes, fibres de collagène et autres glycoprotéines de structure) est l'acteur principal de l'élaboration structurelle du derme.

Les fibres de collagène assurent la solidité du derme. Elles sont très résistantes mais sensibles à certaines enzymes appelées généralement collagénases. Dans le derme, les fibres de collagène sont constituées de fibrilles scellées les unes aux autres, formant ainsi plus de dix types de structures différentes. La structure du derme est en grande partie due à l'enchevêtrement des fibres de collagène tassées. Les fibres de collagène participent à la tonicité de la peau. Les fibres de collagènes sont régulièrement renouvelées mais ce renouvellement diminue avec l'âge ce qui entraîne notamment un amincissement du derme. Il est également admis que des facteurs extrinsèques comme les rayons ultraviolets, le tabac ou certains traitements (Acide rétinoïque et dérivés, glucocorticoïdes, vitamine D et dérivés par exemple) ont également un effet sur la peau et sur son taux de collagène.

Au niveau de la composante dermique de la peau, la glycation intervient principalement dans le derme, sur les fibres de collagène., selon le processus décrit plus haut. La glycation du collagène augmente de façon régulière avec l'âge entraînant une augmentation régulière de la teneur de la peau en produits de glycation.

Sans vouloir introduire une quelconque théorie du vieillissement de la peau, il faut noter que d'autres modifications du collagène qui pourraient également être une conséquence de la glycation, comme une diminution de la dénaturation par la chaleur, une augmentation de la résistance à la digestion enzymatique et une augmentation des pontages intermoléculaires, ont pu être mises en évidence au cours du vieillissement de la peau ( Tanaka S. et col., 1988, J. Mol. Biol., 203, 495-505 ; Takahashi M. et col., 1995, Analytical Biochemistry, 232, 158-162 ). De plus, des modifications dues à la glycation de certains constituants de la membrane basale comme le collagène IV, la laminine et la fibronectine ont pu être mises en évidence (Tarsio JF. et col. , 1985, Diabetes, 34, 477-484 ; Tarsio JF. et col,

1988, Diabetes, 37, 532-539 ; Sternberg M. et col., 1995, C. R. Soc. Biol., 189, 967-985 ).
Ainsi on comprend qu'au cours du vieillissement de la peau les propriétés physico-chimiques du collagène se modifient et ce dernier devient plus difficilement soluble et plus difficilement dégradable.
Ainsi une des composantes de la peau âgée apparaît bien être le collagène glyqué.

On sait très bien que la peau résulte d'une étroite association entre au moins deux compartiments qui la constituent à savoir l'épiderme et le derme. Les interactions entre le derme et l'épiderme sont telles qu'il est raisonnable de penser qu'une modification de l'un peut avoir des conséquences sur l'autre. On peut suspecter que le vieillissement du derme en particulier avec ses phénomènes de glycation ne peut qu'avoir des conséquences sur l'épiderme qui lui est associé. Ainsi au cours du vieillissement cutané, la glycation du collagène doit entraîner des modifications de l'épiderme qui participent nécessairement au vieillissement de l'épiderme.

Ainsi, si la glycation des protéines du derme, particulièrement du collagène entraîne autant de conséquences dommageables au niveau de la peau, des conséquences similaires sont à attendre de la glycation des protéines au niveau des annexes de la peau comme par exemple les ongles et/ou les cheveux et en fait au niveau de tout système protéique.

On comprend donc l'importance qui existe à disposer de produits qui diminuent voire inhibent le phénomène de glycation des protéines.

A cet égard la demanderesse a de manière surprenante et inattendue découvert que les hydroxystilbènes de formule (I) présentent la propriété de diminuer voire inhiber le phénomène de glycation des protéines.

L'invention a donc pour objet l'utilisation dans une composition cosmétique, d'une quantité efficace d'au moins un hydroxystilbène de formule (I), cet hydroxystilbène ou la composition étant destinés à diminuer voire inhiber la glycation des protéines, particulièrement la glycation des protéines de la peau et/ou de ses annexes.

Très particulièrement, l'invention a pour objet l'utilisation dans une composition cosmétique, d'une quantité efficace d'au moins un hydroxystilbène de formule (I), cet hydroxystilbène ou la composition étant destinés à diminuer voire inhiber la glycation des protéines du derme, comme par exemple le collagène, et/ou des ongles et/ou des cheveux, comme par exemple les kératines.

Ainsi l'invention a pour objet l'utilisation dans une composition cosmétique, d'une quantité efficace d'au moins un hydroxystilbène de formule (I), cet hydroxystilbène ou la composition étant destinés à traiter, de manière préventive et/ou curative, les signes du vieillissement de la peau ou de ses annexes liés à la glycation.

Les hydroxystilbènes selon l'invention sont les composés répondant à la formule générale I :

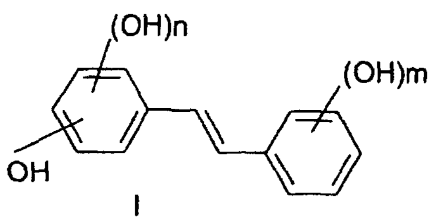

I

dans laquelle n est un nombre entier compris entre 0 et 4 inclusivement et m est un nombre entier compris entre 0 et 5 inclusivement. Ces composés peuvent être sous une forme Cis ou Trans.
Selon l'invention, le terme hydroxystilbène recouvre aussi bien les composés de formule I que leurs dérivés hydroxyalkylés.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse et de carcinogenèse (JP 06024967), ou encore décrits comme antioxydants.

Parmi ces composés le resvératrol (ou 3, 5, 4'-trihydroxystilbène) est particulièrement étudié pour les activités ci-dessus décrites principalement parce qu'il s'agit d'un composé naturel qui se retrouve dans la peau des grains de raisin et dans le vin. A cet égard on peut consulter la revue de Soleas et collaborateurs (Clinical Biochemistry, vol. 30, N°2, pp. 91-113, 1997) qui résume parfaitement l'état des connaissances concernant ce composé et les hydroxystilbènes.
Mais, la capacité des hydroxystilbènes à diminuer voire inhiber le phénomène de glycation n'a jamais été décrite à ce jour.

Parmi les hydroxystilbènes, on peut citer les mono, di, tri, tétra, penta, hexa, hepta, octo, nonahydroxystilbènes, ou encore leurs dérivés hydroxyalkylés.

Selon l'invention les hydroxystilbènes peuvent être utilisés seuls ou en mélanges de toute nature et peuvent être d'origine naturelle ou synthétique.

Les hydroxystilbènes utilisables selon l'invention sont choisis parmi :

le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2, 3',4,4',6-pentahydroxystilbène,
le 2,2',4,4',6,6'-hexahydroxystilbène.

Préférentiellement, on utilise selon l'invention du 3,4',5-trihydroxystilbène ou resvératrol.

Particulièrement l'hydroxystilbène ou la composition le contenant sont utilisés selon l'invention en application topique sur la peau et/ou les ongles et/ou les cheveux.

La quantité d'hydroxystilbène utilisable selon l'invention dépend bien évidemment de l'effet recherché et doit être en une quantité efficace pour diminuer voire inhiber la glycation.

A titre d'exemple, la quantité d'hydroxystilbène utilisable selon l'invention peut aller par exemple de 0,001% à 10% et de préférence de 0,005% à 5% du poids total de la composition.

En outre, la composition de l'invention est utilisée pendant un temps suffisant pour obtenir les effets attendus selon l'invention. Pour donner un ordre de grandeur, cette durée peut être au minimum de 3 semaines, mais peut être aussi de plus de 4 semaines, voire de plus de 8 semaines.

La composition est de préférence destinée à un usage cosmétique ou dermatologique, avantageusement cosmétique.

La composition de l'invention destinée à une application topique contient un milieu physiologiquement acceptable, c'est-à-dire compatible avec la peau y compris le cuir chevelu, ses annexes, les muqueuses et/ou les yeux et peut constituer notamment une composition cosmétique ou dermatologique.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

La composition de l'invention peut constituer par exemple une lotion, un gel, une crème ou un lait, et par exemple une lotion ou un lait de démaquillage ou de nettoyage, un shampooing ou un gel douche.

L'invention a également pour objet un procédé de traitement cosmétique pour traiter les signes du vieillissement liés à la glycation des protéines, particulièrement de la peau et/ou des ongles et/ou des cheveux, caractérisé par le fait que l'on applique sur la peau et/ou les ongles et/ou les cheveux une composition cosmétique comprenant une quantité efficace d'au moins un hydroxystilbène, cet hydroxystilbène ou la composition étant destinés à inhiber la glycation.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral,

sauf indications contraires.

Exemple 1 : Etude de l'effet du resvératrol sur la glycation.

Expérience 1 :

Une solution de sérum albumine bovine à 1 mg/ml en solution dans du tampon phosphate salin (PBS) est incubée à 37°C pendant 14 jours en présence ou en absence de D-ribose à la concentration 10mM ou de resvératrol aux concentrations 1µM et 10µM.
La glycation est évaluée en mesurant la fluorescence des AGEs à λem. = 440 nm émise par chaque échantillon après excitation à λex. = 370 nm.
L'inhibition de la glycation est visualisée par la diminution de la fluorescence comparée à l'échantillon traité avec le sucre seul.
Les résultats sont :

| | resvératrol 1µm | resvératrol 10µM |
|---|---|---|
| % inhibition | 17% | 29,8% |

Le resvératrol présente un effet anti-glycation intéressant dès la concentration de 1 µM.

Expérience 2 :

Le pouvoir antiglycant du resvératrol est évalué sur le cheveu.
La réaction de glycation a été modélisée *in vitro* dans un modèle biochimique utilisant les protéines (kératines) de cheveux humains comme support de glycation par le ribose.

protocole :

Le système d'essai est constitué par des cheveux isolés, incubés dans un tampon phosphate (0,2M, PH= 7.4) contenant 50 mM de ribose (provenance : Sigma).
Le resvératrol dissous dans l'eau de qualité MilliQ est testé aux concentrations $10^{-7}$M, $10^{-6}$M, $10^{-5}$M et $10^{-4}$M.
Le resvératrol est ajouté au système d'essai contenant les cheveux isolés, le ribose et 10% (V/V) d'une solution d'azide de sodium à 0,5% (p/v). L'ensemble est incubé à 50°C à l'abri de la lumière et de l'air pendant 14 jours. Des échantillons témoins, incubés en présence et en absence de ribose, sont testés en parallèle.
Après 14 jours d'incubation, les milieux d'incubation ont été éliminés et les cheveux ont été lavés avec une solution contenant 10 mM de chlorure de guanidium puis dans de l'eau de qualité MilliQ afin d'éliminer toute trace de ribose libre.
Les cheveux ont ensuite été placés dans une solution contenant 50% (v/v) d'éthanol et 50 mM de DTT pendant 15 minutes à 37°C. Après lavage, les échantillons ont été placés dans une solution contenant 10 mM d'acide 5,5'-dithio-bis-2-nitrobenzoique. L'absorbance relative aux résidus cystine libres a été mesurée à 415 nm. Les échantillons ont ensuite été lavés dans une solution d'éthanol à 50% (v/v) puis chauffés pendant 30 minutes à 100°C dans une solution d'hydrazine monohydrate 2M (pH 9.4). Après addition d'une solution de phénylhydrazine à 1 mg/ml dans une solution d'acide acétique 40% (v/v), les échantillons ont été incubés pendant 60°C et l'absorbance relative aux protéines glyquées a été mesurée à 390 nm. Pour chaque échantillon, l'index de glycation a été évalué par le rapport entre l'absorbance à 390 nm et l'absorbance à 415 nm.

$$\text{\% d'inhibition} = \frac{(\text{valeur}_{\text{témoin +sucre}} - \text{valeur}_{\text{produit +sucre}})}{(\text{valeur}_{\text{témoin +sucre}} - \text{valeur}_{\text{témoin-sucre}})} \times 100$$

Les groupes de données (groupe témoin et groupes traités) ont été traités par une analyse de la variance (ANOVA), suivie par un test de Dunett.

Résultats :

| | témoin - ribose | témoin + ribose | R :$10^{-7}$M | R : $10^{-6}$M | R : $10^{-5}$M | R : $10^{-4}$M |
|---|---|---|---|---|---|---|
| indice de glycation | 0,181 | 1,135 | 0,708* | 0,574* | 0,646* | 0,372* |
| Ecart-type | 0,039 | 0,220 | 0,081 | 0,228 | 0,182 | 0,069 |
| % inhibition | - | - | 45 | 59 | 51 | 80 |

R : resvératrol ; ✱ : p< 0,05

Dans ces conditions expérimentales, le composé resvératrol présente une activité anti-glycation de 80% à la concentration $10^{-4}$ M.

**Revendications**

1. Utilisation cosmétique dans une composition cosmétique d'une quantité efficace d'au moins un hydroxystilbène de formule (I)

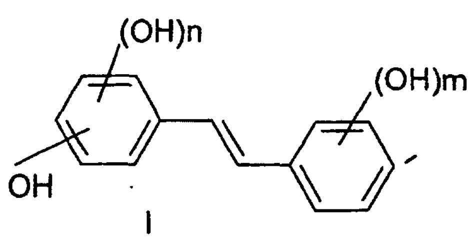

I

dans laquelle n est un nombre entier compris entre 0 et 4 inclusivement et m est un nombre entier compris entre 0 et 5. inclusivement, pour diminuer la glycation des protéines de la peau et/ou des ongles et/ou des cheveux.

2. Utilisation selon la revendication précédente, **caractérisée par le fait que** les protéines de la peau sont les protéines du derme.

3. Utilisation selon la revendication précédente, pour diminuer la glycation du collagène.

4. Utilisation selon la revendication 1, **caractérisée par le fait que** les protéines des ongles et/ou des cheveux sont les kératines.

5. Utilisation selon l'une quelconque des revendications précédentes pour traiter, de manière préventive et/ou curative, les signes du vieillissement des ongles et/ou des cheveux liés à la glycation.

6. Utilisation selon l'une quelconque des revendications précédentes, **caractérisée par le fait que** l'hydroxystilbène est choisi parmi :

le 4'-hydroxystilbène,
le 2',4'-dihydroxystilbène,
le 3',4'-dihydroxystilbène,
le 4,4'-dihydroxystilbène,
le 2',4',4-trihydroxystilbène,
le 3',4',4-trihydroxystilbène,
le 2,4,4'-trihydroxystilbène,
le 3,4,4'-trihydroxystilbène,
le 3,4',5-trihydroxystilbène,
le 2',3,4-trihydroxystilbène,
le 2,3',4-trihydroxystilbène,
le 2',2,4'-trihydroxystilbène,
le 2,4,4',5-tétrahydroxystilbène,
le 2',3,4',5-tétrahydroxystilbène,
le 2,2',4,4'-tétrahydroxystilbène,
le 3,3',4',5-tétrahydroxystilbène,
le 2,3',4,4'-tétrahydroxystilbène,
le 3,3',4,4'-tétrahydroxystilbène,
le 3,3', 4',5, 5'-pentahydroxystilbène,
le 2,2',4,4',6-pentahydroxystilbène,
le 2,3',4,4',6-pentahydroxystilbène,
le 2,2',4,4',6,6'-hexahydroxystilbène.

**7.** Utilisation selon l'une quelconque des revendications précédentes, **caractérisée par le fait que** l'hydroxystilbène est du 3,4',5-trihydroxystilbène ou resvératrol.

**8.** Utilisation selon l'une quelconque des revendications précédentes, **caractérisée par le fait que** l'hydroxystilbène est présent en une quantité allant de 0,001% à 10% du poids total de la composition

**9.** Utilisation selon la revendication précédente, **caractérisée par le fait que** l'hydroxystilbène est présent en une quantité allant de 0,005.% à 5 % du poids total de la composition.

**10.** Procédé de traitement cosmétique pour traiter les signes du vieillissement liés à la glycation des protéines des ongles et/ou des cheveux, **caractérisé par le fait que** l'on applique sur les ongles et/ou les cheveux une composition cosmétique comprenant une quantité efficace d'au moins un hydroxystilbène de formule (I)

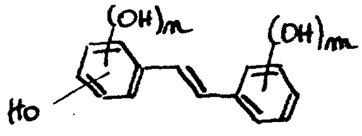

dans laquelle m est un nombre entier compris entre 0 et 4 inclusivement et m est un nombre entier compris entre 0 et 5 inclusivement, cet hydroxystilbène ou la composition étant destinés à inhiber la glycation.

## Patentansprüche

**1.** Kosmetische Verwendung mindestens eines Hydroxystilben der folgenden Formel (I) in einer wirksamen Menge in einer kosmetischen Zusammensetzung:

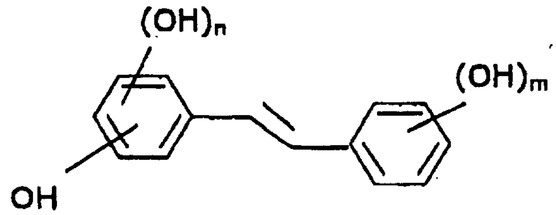

(I),

worin n 0 oder eine ganze Zahl von 1 bis 4 und m 0 oder eine ganze Zahl von 1 bis 5 bedeutet, um die Glykation von Proteinen der Haut und/oder der Nägel und/oder der Hautanhangsgebilde zu inhibieren.

**2.** Verwendung nach dem vorhergehenden Anspruch, **dadurch gekennzeichnet, dass** es sich bei den Proteinen der Haut um Proteine der Dermis handelt.

**3.** Verwendung nach dem vorhergehenden Anspruch, um die Glykation des Collagen zu inhibieren.

**4.** Verwendung nach Anspruch 1, **dadurch gekennzeichnet, dass** es sich bei den Proteinen der Nägel und/oder der Haare um Keratine handelt.

**5.** Verwendung nach einem der vorhergehenden Ansprüche zur kurativen und/oder präventiven Behandlung der mit der Glykation zusammenhängenden Anzeichen der Alterung der Nägel und/ oder der Haare.

**6.** Verwendung nach einem der vorhergehenden Ansprüche, **dadurch gekennzeichnet, dass** das Hydroxystilben ausgewählt ist unter:

4'-Hydroxystilben,
2',4'-Dihydroxystilben,
3',4'-Dihydroxystilben,
4,4'-Dihydroxystilben,
2',4',4-Trihydroxystilben,
3',4',4-Trihydroxystilben,
2,4,4'-Trihydroxystilben,
3,4,4'-Trihydroxystilben,
3,4',5-Trihydroxystilben,
2',3,4-Trihydroxystilben,
2,3',4-Trihydroxystilben,
2',2,4'-Trihydroxystilben,
2,4,4', 5-Tetrahydroxystilben,
2',3,4',5-Tetrahydroxystilben,
2,2',4,4'-Tetrahydroxystilben,
3,3',4',5-Tetrahydroxystilben,
2,3',4,4'-Tetrahydroxystilben,
3,3',4,4'-Tetrahydroxystilben,
3,3',4',5,5'-Pentahydroxystilben,
2,2',4,4',6-Pentahydroxystilben,
2,3',4,4',6-Pentahydroxystilben,
2,2',4,4',6,6'-Hexahydroxystilben.

**7.** Verwendung nach einem der vorhergehenden Ansprüche, **dadurch gekennzeichnet, dass** es sich bei dem Hydroxystilben um das 3,4',5-Trihydroxystilben oder Resveratrol handelt.

**8.** Verwendung nach einem der vorhergehenden Ansprüche, **dadurch gekennzeichnet, dass** das Hydroxystilben in einer Menge von 0,001 bis 10 % des Gesamtgewichts der Zusammensetzung vorliegt.

**9.** Verwendung nach dem vorhergehenden Anspruch, **dadurch gekennzeichnet, dass** das Hydroxystilben in einer Menge von 0,005 bis 5 % des Gesamtgewichts der Zusammensetzung vorliegt.

**10.** Verfahren zur kosmetischen Behandlung der mit der Glykation von Proteinen der Nägel und/oder der Haare zusammenhängenden Anzeichen der Hautalterung, **dadurch gekennzeichnet, dass** auf die Nägel und/oder die Haare eine kosmetische Zusammensetzung aufgetragen wird, die mindestens ein Hydroxystilben der Formel (I)

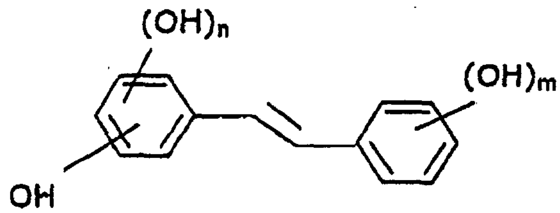

(I),

worin n 0 oder eine ganze Zahl von 1 bis 4 und m 0 oder eine ganze Zahl von 1 bis 5 bedeutet, in einer wirksamen Menge enthält, wobei das Hydroxystilben oder die Zusammensetzung dazu vorgesehen sind, die Glykation zu inhibieren.

## Claims

**1.** Cosmetic use, in a cosmetic composition, of an effective amount of at least one hydroxystilbene of formula (I)

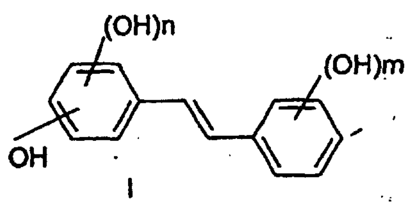

I

in which n is an integer of between 0 and 4 inclusively and m is an integer of between 0 and 5 inclusively, for reducing the glycation of proteins of the skin and/or the nails and/or the hair.

**2.** Use according to the preceding claim, **characterized in that** the skin proteins are dermal proteins.

**3.** Use according to the preceding claim, for reducing the glycation of collagen.

**4.** Use according to Claim 1, **characterized in that** the proteins of the nails and/or the hair are keratins.

**5.** Use according to any one of the preceding claims, for preventively and/or curatively treating the signs of ageing of the nails and/or the hair which are associated with glycation.

**6.** Use according to any one of the preceding claims, **characterized in that** the hydroxystilbene is chosen from:

4'-hydroxystilbene,
2',4'-dihydroxystilbene,
3',4'-dihydroxystilbene,
4,4'-dihydroxystilbene,
2',4',4-trihydroxystilbene,
3',4',4-trihydroxystilbene,
2,4,4'-trihydroxystilbene,
3,4,4'-trihydroxystilbene,
3,4',5-trihydroxystilbene,
2',3,4-trihydroxystilbene,
2,3',4-trihydroxystilbene,
2',2,4'-trihydroxystilbene,
2,4,4',5-tetrahydroxystilbene

2',3,4',5-tetrahydroxystilbene,
2,2',4,4'-tetrahydroxystilbene,
3,3',4',5-tetrahydroxystilbene,
2,3',4,4'-tetrahydroxystilbene,
3,3',4,4'-tetrahydroxystilbene,
3,3',4',5,5'-pentahydroxystilbene,
2,2',4,4',6-pentahydroxystilbene,
2,3'4,4',6-pentahydroxystilbene,
2,2',4,4',6,6'-hexahydroxystilbene.

**7.** Use according to any one of the preceding claims, **characterized in that** the hydroxystilbene is 3,4',5-trihydroxystilbene, or resveratrol.

**8.** Use according to any one of the preceding claims, **characterized in that** the hydroxystilbene is present in an amount ranging from 0.001% to 10% relative to the total weight of the composition.

**9.** Use according to the preceding claim, **characterized in that** the hydroxystilbene is present in an amount ranging from 0.005% to 5% relative to the total weight of the composition.

**10.** Cosmetic treatment process for treating the signs of ageing associated with the glycation of proteins of the nails and/or the hair, **characterized in that** a cosmetic composition comprising an effective amount of at least one hydroxystilbene of formula (I)

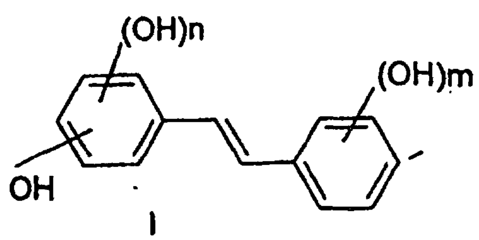

I

in which n is an integer of between 0 and 4 inclusively and m is an integer of between 0 and 5 inclusively, is applied to the nails and/or the hair, this hydroxystilbene or the composition being intended to inhibit glycation.